# EUROPEAN PATENT APPLICATION

(11) **EP 4 442 284 A1**
(43) Date of publication of application: **09.10.2024**
(21) Application number: 24152275.4
(22) Date of filing: 17.01.2024
(51) Int. Cl.: A61K 49/00

(54) **STAINING AGENT FOR GASTROINTESTINAL MUCOSA AND PREPARATION METHOD THEREFOR**

(30) Priority: 18.01.2023 CN 202310095867
(71) Applicant: Beijing Compont Medical Devices Co., Ltd, Beijing 100176 (CN)
(72) Inventor: XIA, Yingjing, Beijing (CN); LIU, Xiaochen, Beijing (CN); DUAN, Yingjie, Beijing (CN); ZHANG, Jianguo, Beijing (CN); SHEN, Wei, Beijing (CN)
(74) Representative: LLR

(57) **Abstract**

The present invention provides a staining agent for gastrointestinal mucosa, including acid violet 43. Compared with existing commercially available staining agent products, the staining agent for gastrointestinal mucosa provided by the present invention has better safety, rinsing performance and stability. It is not susceptible to oxidation and decomposition under conventional conditions, and may provide a longer shelf life. Meanwhile, its staining effect is better than those of commercially available staining agent products at a low concentration.

## Description

### Technical Field

The present invention relates to the field of medical staining agents, and in particular to a staining agent for gastrointestinal mucosa and a preparation method therefor.

### Background Art

A staining agent for gastrointestinal mucosa is a kind of auxiliary products used for improving the examination effect on the basis of conventional endoscopy. When in use, the staining agent is sprayed to a certain site of gastrointestinal mucosa, and then observed by endoscopy. Compared with a normal tissue, for a focus site, a lesion site sprayed with a staining agent is clearer in focus shape and range, and is more obviously recognized by naked eyes, which significantly increases a detection rate of a lesion, and greatly reduces the workload of a doctor.

At present, common staining agents on the market mainly include: indigo carmine, methylene blue, Lugol's iodine, etc. The principle of action of the indigo carmine staining agent is to perform staining comparison by means of physical deposition. However, the indigo carmine is blue, and is greatly different from the digestive tract in color. After staining, there is a strong discrepancy for distinctive visual sense, causing problems such as an unclear staining visual field. Methylene blue staining is to make a mucosa turn blue by absorbing the methylene blue by the mucosa. Its disadvantages are that the methylene blue cannot be rinsed after being used for staining, and re-staining is needed if staining is unsatisfactory; and after the methylene blue is absorbed, human hemoglobin may become methemoglobin, and some patients may have adverse reactions such as nausea, abdominal pain or urticaria. A required concentration of the Lugol's iodine of 5 % is too high; the Lugol's iodine needs to be freshly prepared in actual use; its application is cumbersome; and its concentration cannot be accurate. Meanwhile, the indigo carmine staining agent is unstable in properties, and may undergo a redox reaction with oxygen in air at a room temperature.

In view of the above, the present invention is proposed now.

### Summary of the Invention

A purpose of the present invention is to provide a staining agent for gastrointestinal mucosa with better stability, safety and use effect than existing commercially available similar products.

In order to achieve the above purpose, the present invention provides a staining agent for gastrointestinal mucosa, including acid violet 43.

The acid violet 43 used in the present invention is also called Violet No. 2 in China.

Preferably or optionally, the concentration of the acid violet 43 is 0.01-5 %.

Preferably or optionally, the staining agent further includes allure red.

Preferably or optionally, the concentration of the acid violet 43 is 0.02-5 %, and the concentration of the allure red is 0.01-2.5 %.

Preferably or optionally, the concentration ratio of the acid violet 43 to the allure red is 2: 1. Preferably or optionally, the staining agent further includes an antioxidant.

Preferably or optionally, the antioxidant is one or more selected from the group consisting of citric acid, ascorbic acid, tea polyphenol, propyl gallate, tert-butyl hydroquinone, butylated hydroxytoluene and butyl hydroxyanisole.

Preferably or optionally, the concentration of the antioxidant in the staining agent is 0.01-5 %.

In another aspect, the present invention further provides a preparation method for the above staining agent for gastrointestinal mucosa, including the following steps:
(1) dissolving the components into purified water according to a ratio, and performing uniform stirring to obtain a mixture; and
(2) filling the mixture into a sealed bottle for packaging to obtain a staining agent product for gastrointestinal mucosa.

Preferably or optionally, the sealed bottle is a brown sealed bottle.

### Beneficial Effects

Compared with existing commercially available staining agent products, the staining agent for gastrointestinal mucosa provided by the present invention has better safety, rinsing performance and stability. It is not susceptible to oxidation and decomposition under conventional conditions, and may provide a longer shelf life. Meanwhile, its staining effect is better than those of commercially available staining agent products at a low concentration.

### Brief Description of the Drawings

FIG. 1 is a diagram of the staining effect of the staining agent provided by Example 1 in Effective Example 1;
FIG. 2 is a diagram of the staining effect of the staining agent provided by Comparative Example 1 in Effective Example 1;
FIG. 3 is a diagram of the staining effect of the staining agent provided by Comparative Example 2 in Effective Example 1;
FIG. 4 is a diagram of comparison in state before and after staining and rinsing of the staining agent provided by Comparative Example 1 in Effective Example 2;
FIG. 5 is a diagram of comparison in state before and after staining and rinsing of the staining agent provided by Example 1 in Effective Example 2;
FIG. 6 is a diagram of comparison in state before and after staining and rinsing of the staining agent provided by Example 2 in Effective Example 2;
FIG.7 is a diagram of experimental results of the group in Example 1 in Effective Example 3; and
FIG. 8 is a diagram of experimental results of the group in Comparative Example 1 in Effective Example 3.

### Detailed Description of the Invention

In order to facilitate the understanding of the present invention, the present invention will be more fully described with reference to the accompanying drawings and preferred examples below in more detail, but the scope of protection of the present invention is not limited in the following examples. Unless defined otherwise, all generic terms used below have the same meaning as commonly understood by those skilled in the art. The generic terms used herein are for the purpose of describing particular examples only and are not intended to limit the scope of protection of the present invention. Unless otherwise specified, all the raw materials, reagents, instruments, equipment, etc. used in the present invention may be purchased from the market or may be prepared by existing methods.

### Example 1

The example of the present invention provides a staining agent for gastrointestinal mucosa.

The staining agent for gastrointestinal mucosa was prepared by the following method:
Acid violet 43 and allure red were weighed in a sterile room; purified water was then added to a fixed volume of 100 mL to make a concentration of the acid violet 43 be 0.1 % and a concentration of the allure red be 0.05 %; and then, sodium citrate was added to make its concentration be 0.1 %. The prepared staining agent for gastrointestinal mucosa was filled into a 50 mL penicillin bottle, and sterilized with steam at 121 °C for 30 min to obtain a staining agent product for gastrointestinal mucosa.

### Example 2

The example of the present invention provides a staining agent for gastrointestinal mucosa.

The staining agent for gastrointestinal mucosa was prepared by the following method:
Acid violet 43 and sodium citrate were weighed in a sterile room; and purified water was then added to a fixed volume of 100 mL to make a concentration of the acid violet 43 be 0.1 % and a concentration of the sodium citrate be 0.1 %.

The prepared staining agent for gastrointestinal mucosa was filled into a 50 mL penicillin bottle, and sterilized with steam at 121 °C for 30 min to obtain a staining agent product for gastrointestinal mucosa.

### Example 3

The example of the present invention provides a staining agent for gastrointestinal mucosa.

The staining agent for gastrointestinal mucosa was prepared by the following method:
Acid violet 43 and allure red were weighed in a sterile room; purified water was then added to a fixed volume of 100 mL to make a concentration of the acid violet 43 be 0.02 % and a concentration of the allure red be 0.01 %; and then, sodium citrate was added to make its concentration be 0.1 %. The prepared staining agent for gastrointestinal mucosa was filled in a 50 mL penicillin bottle, and sterilized with steam at 121 °C for 30 min to obtain a staining agent product for gastrointestinal mucosa.

### Example 4

The example of the present invention provides a staining agent for gastrointestinal mucosa.

The staining agent for gastrointestinal mucosa is prepared by the following steps:
Acid violet 43 and allure red were weighed in a sterile room; purified water was then added to a fixed volume of 100 mL to make a concentration of the acid violet 43 be 2 % and a concentration of the allure red be 1 %; and then, sodium citrate was added to make a concentration of the sodium citrate be 0.1 %.

The prepared staining agent for gastrointestinal mucosa was filled into a 50 mL penicillin bottle, and sterilized with steam at 121 °C for 30 min to obtain a staining agent product for gastrointestinal mucosa.

### Comparative Example 1

This comparative example provides a staining agent for gastrointestinal mucosa.

Indigo carmine and sodium citrate were weighed in a sterile room; and purified water was then added to a fixed volume of 100 mL to make a concentration of the indigo carmine be 0.2 % and a concentration of the sodium citrate be 0.1 %.

The prepared staining agent for gastrointestinal mucosa was filled into a 50 mL penicillin bottle, and sterilized with steam at 121 °C for 30 min to obtain a staining agent product for gastrointestinal mucosa.

### Comparative Example 2

This comparative example provides a staining agent for gastrointestinal mucosa.

The staining agent for gastrointestinal mucosa was prepared by the following method:
Acid violet 43 and allure red were weighed in a sterile room; purified water was then added to a fixed volume of 100 mL to make a concentration of the acid violet 43 be 0.08 % and a concentration of the allure red be 0.05 %; and then, sodium citrate was added to make its concentration be 0.1 %.

The prepared staining agent for gastrointestinal mucosa was filled into a 50 mL penicillin bottle, and sterilized with steam at 121 °C for 30 min to obtain a staining agent product for gastrointestinal mucosa.

### Effective Example 1

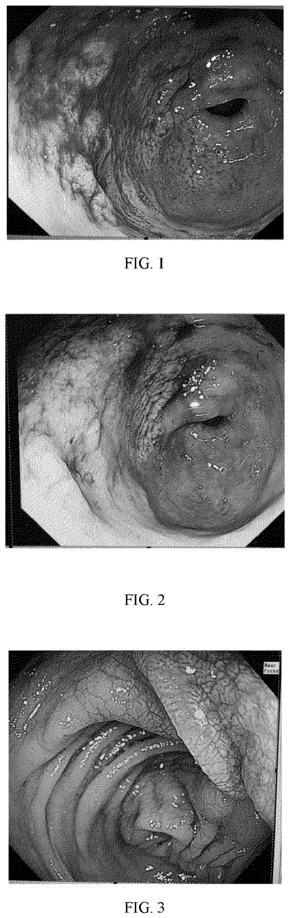

Bottles charged with the staining agent products for gastrointestinal mucosa prepared by Example 1 and Comparative Examples 1-2 were wiped with medical alcohol and then transferred into an operating room. Then, after a disease site needed to be stained was rinsed with normal saline, the staining agents were sprayed to the disease site by cooperating with an endoscopic spraying device, and staining was observed under a gastroscopic lens. Results are shown in FIG. 1 and FIG. 2. Among them, the staining result of Example 1 is shown in FIG. 1; the staining result of Comparative Example 1 is shown in FIG. 2; and the staining result of Comparative Example 2 is shown in FIG. 3.

From a comparison on FIGs. 1-3, it can be seen that the staining agent for gastrointestinal mucosa provided by Example 1 has a better staining effect on the disease site, and has a stronger stereo sense and more obvious details; while the disease site stained by the staining agent provided by Comparative Example 2 has a dark color under a gastroscope due to an incorrect ratio of red and blue pigments in the staining agent, which is not conducive to observing a mucosal visual field.

### Effective Example 2

1 mL of the staining agents for gastrointestinal mucosa prepared by Examples 1-2 and Comparative Example 1 were respectively sprayed to clean pig large intestines which were processed and cut into a length of 5 cm. After 30 s of staining, 10 mL of normal saline was used to rinse the staining sites, and the effects after rinsing were observed.

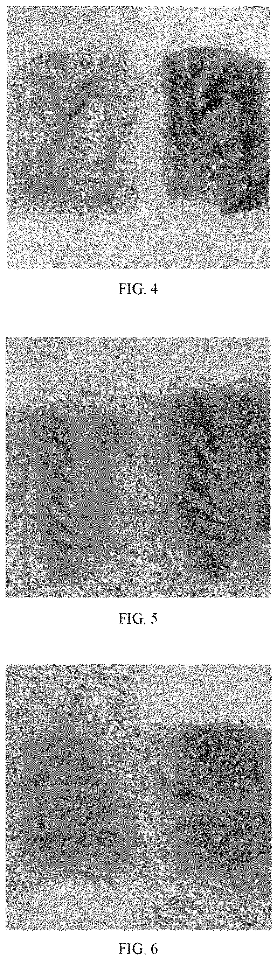

Experimental results are shown in FIGs. 4-6, wherein the left side of each drawing is a sample before staining, and the right side is the sample after staining and rinsing.

It can be seen from FIGs. 4-6 that there are still more staining agent residues after staining and rinsing of the staining agent in the group of Comparative Example 1; while the groups of Example 1 and Example 2 have similar colors after rinsing to those before treatment, and the staining agent residues are much less than those in the group of Comparative Example 1. It can be seen that the staining agents for gastrointestinal mucosa provided by the examples of the present invention have better rising performance than commercially available indigo carmine staining agent products.

### Effective Example 3

Staining agents for gastrointestinal mucosa with and without sodium citrate (an antioxidant) were prepared according to the technical solutions of Example 1 and Comparative Example 1, respectively.

The prepared staining agents in various groups were placed in lights and at a room temperature for 72 h, and color changes of the staining agents without adding the antioxidants in various groups compared with the staining agents added with the antioxidants were observed.

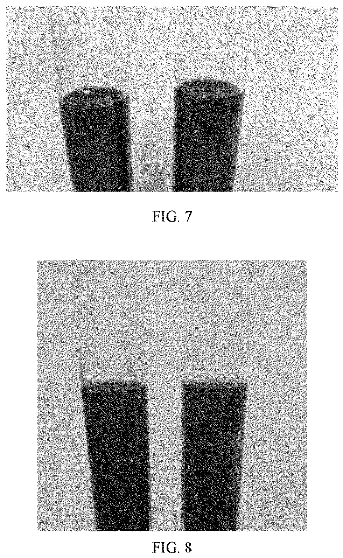

Experimental results of the group of Example 1 are shown in FIG. 7; and experimental results of the group of Comparative Example 1 are shown in FIG. 8.

It can be seen from FIG. 7 and FIG. 8, there is no significant change between the staining agent without the antioxidant (the right tube) and the staining agent with the antioxidant (the left tube) in the group of Example 1. In the group of Comparative Example 1, the staining agent without the antioxidant (the right tube) obviously fades compared with the staining agent with the antioxidant (the left tube). Therefore, the staining agents for gastrointestinal mucosa provided by the examples of the present invention have better stability than commercially available indigo carmine staining agent products.

For safety, acid violet 43 has been published in International Journal of Toxicology in 2010. It has no abnormal report in acute oral, short-term skin, subchronic skin, heredity and other aspects, so its safety test data are sufficient to support its inclusion in drug and cosmetic uses.

According to relevant contents in the report provided by EFSA in 2009, an allowable daily intake (ADI) of the allure red is 7 mg/kg.bw/day; while for a comparison, an ADI of the indigo carmine is 5 mg/kg.bw/day, and meanwhile, there is no adverse event in the report on its toxicity.

In another aspect, for commercially available products, a concentration of methylene blue staining agent is generally 0.3 %, a concentration of the Lugol's iodine is generally 5 %, and a concentration of the indigo carmine staining agent is generally 0.2 %. Therefore, through the above experimental results, in the technical solution described in the present invention that the acid violet 43 is used to prepare the staining agent for gastrointestinal mucosa, its staining effect is better than those of commercially available staining agent products at a low concentration (for commercially available staining agents, the staining effect of the indigo carmine staining agent is better than those of the methylene blue staining agent and the lugol's iodine staining agent). After being compounded with the allure red, the acid violet 43 is further improved in staining effect.

To sum up, compared with the existing commercially available staining agent products, the staining agent for gastrointestinal mucosa provided by the present invention has better safety, rinsing performance and stability. It may provide a longer shelf life. Meanwhile, its staining effect is better than those of commercially available staining agent products at a low concentration.

Finally, it is to be noted that: the above examples are only used to explain the technical solutions of the present invention and shall not be construed as limitations. Although the present invention has been described in detail with respect to the previously described examples, it should be appreciated by those of ordinary skill in the art, the technical solutions described in the examples may be still modified, or part of its technical features may be substituted with equivalents; and such modifications or substitutions do not deviate the nature of the technical solutions from the spirit and scope of the technical solutions of the various examples in the present invention.

## Claims

1. A staining agent for gastrointestinal mucosa, comprising acid violet 43.

2. The staining agent according to claim 1, wherein the concentration of the acid violet 43 is 0.01-5 %.

3. The staining agent according to claim 1, further comprising allure red.

4. The staining agent according to claim 3, wherein the concentration of the acid violet 43 is 0.02-5 %, and the concentration of the allure red is 0.01-2.5 %.

5. The staining agent according to claim 4, wherein the concentration ratio of the acid violet 43 to the allure red is 2: 1.

6. The staining agent according to any one of claims 1-5, further comprising an antioxidant.

7. The staining agent according to claim 6, wherein the antioxidant is one or more selected from the group consisting of citric acid, ascorbic acid, tea polyphenol, propyl gallate, tert-butyl hydroquinone, butylated hydroxytoluene and butyl hydroxyanisolee.

8. The staining agent according to claim 7, wherein the concentration of the antioxidant in the staining agent is 0.01-5 %.

9. A preparation method for the staining agent for gastrointestinal mucosa according to any one of claims 1-8, comprising the following steps:
(1) dissolving the components into purified water according to a ratio, and performing uniform stirring to obtain a mixture; and
(2) filling the mixture into a sealed bottle for packaging to obtain a staining agent product for gastrointestinal mucosa.

10. The preparation method according to claim 9, wherein the sealed bottle is a brown sealed bottle.
